# EUROPÄISCHE PATENTSCHRIFT

(11) **EP 1 382 353 B1**
(45) Veröffentlichungstag und Bekanntmachung des Hinweises auf die Patenterteilung: **25.02.2009**
(21) Anmeldenummer: 03016184.8
(22) Anmeldetag: 17.07.2003
(51) Int. Cl.: A61L 2/04, A61L 2/00, A61L 2/26, A23L 3/02, A23L 3/10

(54) **Verfahren und Vorrichtung zum Sterilisieren eines in einer Verpackung abgepackten Produktes**
Process and apparatus for sterilization of products in packages
Procédé et dispositif pour la stérilisation de produits en emballage

(30) Priorität: 19.07.2002 DE 10233095
(43) Veröffentlichungstag der Anmeldung: 21.01.2004
(73) Patentinhaber: SIG Technology Ltd., 8212 Neuhausen am Rheinfall (CH)
(72) Erfinder: Bömer, Hans, 40699 Erkrath (DE); Wolters, Michael, Dr., 52525 Heinsberg (DE)
(74) Vertreter: Cohausz & Florack

(56) Entgegenhaltungen:
- EP-A- 0 891 781
- EP-A- 1 159 971
- WO-A-01/26488
- WO-A-90/06692
- GB-A- 386 035

## Beschreibung

Die Erfindung betrifft ein Verfahren und eine Vorrichtung zum Sterilisieren eines in einer im Wesentlichen formstabilen Verpackung abgepackten Produktes, wobei die aus Produkt und Verpackung bestehenden Packungen einer Wärmebehandlung bei einer bestimmten Temperatur über eine bestimmte Dauer unterzogen werden und wobei das Produkt aus einer Flüssigkeit und stückigen Anteilen besteht, wobei in einen kontinuierlich arbeitenden Autoklaven eine Mehrzahl von Packungen eingebracht werden und wobei die Packungen nach dem Sterilisierprozess aus dem Autoklaven ausgeschleust werden.

Es ist seit langem bekannt, portioniert abgepackte Produkte wie beispielsweise Lebensmittel zur Haltbarmachung einem sog. Autoklavierprozess zu unterziehen. Hierbei werden beispielsweise Konservendosen, in welche vorher ein Lebensmittelprodukt abgepackt worden ist, für eine bestimmte Dauer in einem Autoklaven einer bestimmten Temperatur ausgesetzt, um die in der Packung befindlichen Keime zuverlässig abzutöten. Konservendosen haben sich für den genannten Zweck insbesondere deshalb bewährt, weil sie unempfindlich gegenüber den in einem Autoklaven herrschenden Bedingungen (hohe Temperatur, hohe Feuchte) sind.

Werden Lebensmittel oder andere zu sterilisierende Produkte jedoch nicht in Konserven, sondern in anderen Verpackungen auf den Markt gebracht, so muss - mit relativ hohem Aufwand - zunächst die Verpackung sterilisiert werden, dann das Produkt unter sterilen Bedingungen in die Verpackung gefüllt werden und schließlich die Verpackung aseptisch geschlossen werden. Auch die so hergestellten Karton/Kunststoff-Verbundpackungen sind in der Lage, Produkte wie beispielsweise Milch oder püriertes Gemüse über einen längeren Zeitraum aufzunehmen.

Die vorliegende Erfindung befasst sich jedoch mit dem Sterilisieren eines aus einer Flüssigkeit und stückigen Anteilen bestehenden Produkts, welches in im Wesentlichen formstabilen, quaderförmigen Packungen, wie beispielsweise in Karton/Kunststoff-Verbundpackungen mit oder ohne Fensterausschnitt abgefüllt worden ist. Hierbei reicht es nicht aus, das Karton/Kunststoff-Verbundmaterial so auszugestalten, dass es den Bedingungen im Autoklaven gerecht wird, da die ungleiche Verteilung von Flüssigkeit und stückigen Anteilen im Inneren der Packung eine homogene, d. h. gleichmäßige Erwärmung verhindert. Als stückige Anteile sind hier beispielsweise Erbsen, Mais, Tomaten, Bohnen oder Fleisch zu nennen, welche in einer Soße oder in einem Sud angeboten werden. Gleiches gilt für Tiernahrungsprodukte. Die vorgenannten Produkte werden dabei regelmäßig mit gar keinem oder nur einem möglichst geringen Luftanteil verpackt, um das Füllvolumen der Verpackungen optimal ausnutzen zu können.

Aus der DE 100 26 539 A1 ist es für sich bereits bekannt, in flexiblen Packungen, wie beispielsweise Standbodenbeuteln, abgepackte Produkte während des Sterilisiervorgangs im Autoklaven in ihrer Lage zu bewegen, wodurch aufgrund der in solchen Packungen vorhandenen Luftblase ein "Schwappen" des Produktes im Inneren der Packungen bewirkt, was zu einer Verteilung des Produktes und damit zu einer gleichmäßigeren Erwärmung führt.

Aus der EP 0 609 087 A1 ist es für sich bereits bekannt, im Inneren eines Autoklaven eine Bewegung der zu sterilisierenden Produkte zu erzeugen, indem diese auf einem Schüttel- oder Vibrationstisch angeordnet sind. Auch werden hier bereits eine Pendelbewegung oder die Bewegung nach einem Sägezahnmuster bzw. ein exzentrisches Rotieren als alternative Bewegungsformen beschrieben.

Die WO-A-90/06692 betrifft ein Verfahren und eine Vorrichtung zum kontinuierlichen Sterilisieren von Konservendosen. Die Konservendosen werden angetrieben von einer Transporteinrichtung durch einen Autoklaven bewegt und dabei in eine Rotation um ihre Längsachse versetzt. Diese Rotationsbewegung wird entweder durch Abrollen der Konservendosen auf einer Unterlage oder durch hierfür vorgesehene Walzen, die dann ihrerseits auf einer Unterlage abrollen, hervorgerufen. Während des Transports durch den Autoklaven werden die Konservendosen zunächst aufgeheizt, dann für eine bestimmte Zeit auf einer Sterilisierungstemperatur gehalten und anschließend wieder abgekühlt, wobei für die Abkühlung eine Berieselungseinheit vorgesehen sein kann.

Aus der GB-A-386,035 geht ein Verfahren mit einer Vorrichtung zum Sterilisieren von zylinderförmigen Dosen hervor. Die bekannte Vorrichtung weist einen rotationssymmetrischen Autoklaven mit einer sich vertikal in seinem Inneren erstreckenden und mehrmals unterbrochenen Spirale auf. Die Dosen werden nacheinander von oben in den Autoklaven eingebracht und wandern langsam entlang der Spirale nach unten, wo sie nach erfolgter Sterilisierung wieder aus dem Autoklaven austreten. Der Transport der Dosen erfolgt nicht allein aufgrund der Schwerkraft, sondern durch mehrere Arme einer entlang der Längsachse des Autoklaven angeordneten, drehbaren Welle. Die einzelnen Arme sind in vertikaler Richtung jeweils in Abständen auf der Welle angeordnet, die der vertikalen Erstreckung einer halben Spiralwicklung entspricht. Sie rotieren angetrieben von der Welle jeweils im Bereich einer Unterbrechung der Spirale. Bei einer vollständigen Umdrehung eines Arms fördert dieser eine Dose entlang einer halben Spiralwicklung weiter nach unten, wo die Dose dann von einem weiteren Arm erfasst und von diesem um eine weitere, halbe Spiralwicklung weiter nach unten transportiert wird.

Die WO-A-01/26488 betrifft ein Verfahren und eine Vorrichtung zum diskontinuierlichen Sterilisieren von verpackten Lebensmitteln innerhalb eines Autoklaven. Der Autoklav als solches weist an zwei einander gegenüberliegenden Seiten je eine hermetisch verschließbare Öffnung auf. Dabei dient eine der Öffnungen zur Zuführung von zu sterilisierenden Packungen, während die gegenüberliegende Öffnung zur Entnahme der sterilisierten Packungen dient. Nachdem ein Bündel von mehreren Packungen in den Autoklaven eingebracht worden ist und sich auf einem von Walzen gebildeten Transportband befindet, wird das Bündel relativ zu dem Transportband fixiert und der Autoklav hermetisch verschlossen. Das Bündel befindet sich nun innerhalb einer von zwei parallelen Ringen definierten Trommel, welche während des Autoklavierprozesses um seine Längsachse gedreht wird. Nach Beendigung des Sterilisationsprozesses wird das Bündel in seiner Ausgangslage durch die zweite Öffnung aus dem Autoklaven entfernt.

Die EP-A-0 891 781 offenbart ein kontinuierliches Sterilisationsverfahren von Lebensmitteln in flexiblen Folienverpackungen innerhalb eines Autoklaven sowie eine Vorrichtung zur Durchführung eines solchen Verfahrens. Die bekannte Vorrichtung weist zwei durch einen Dampfraum voneinander getrennte und flüssigkeitsgefüllte Siphons auf. Verfahrensgemäß ist vorgesehen, die einzelnen Packungen in sogenannte Trays einzubringen, die nacheinander an einer endlos durch die gesamte Vorrichtung geführten Kette befestigt sind. In Transportrichtung der Kette werden die Packungen zunächst durch die Wassersäule des ersten Siphons hindurchgeführt, wobei die Packungen aufgeheizt werden. Anschließend gelangen die Packungen in den unter einem Überdruck stehenden Dampfraum, in dem die Sterilisationstemperatur herrscht. Nachdem die Packungen durch den Dampfraum geführt worden sind, gelangen sie in eine zweite Wassersäule, die der Abkühlung der Packungen dient. Entsprechend der gewählten Art der Befestigung der Trays an der Kette ergibt es sich, dass die Packungen zwischenzeitlich auf den Kopf und wieder zurückgedreht werden, ohne dass dies hier einem besonderen Zweck dienen würde.

Die EP-A-1 159 971 zeigt ein diskontinuierliches Sterilisationsverfahren von Lebensmitteln in Standbodenbeuteln innerhalb eines Autoklaven. Die Standbodenbeutel liegen in Stützeinrichtungen (Trays), die während der Sterilisation gekippt werden. Es handelt sich letztlich um eine diskontinuierliche Sterilisierung, die von einer Rüttelbewegung begleitet wird.

Davon ausgehend liegt der Erfindung die Aufgabe zu Grunde, ein Verfahren und eine Vorrichtung zum Sterilisieren eines in einer im Wesentlichen formstabilen Verpackung abgepackten Produktes so auszugestalten und weiterzubilden, dass auch solche Produkte, die neben einer Flüssigkeit auch stückige Anteile enthalten und in im wesentlichen quaderförmigen Verpackungen abgepackt sind, zuverlässig sterilisiert werden können, ohne die zu sterilisierenden Produkte zu schädigen.

Verfahrensmäßig erfolgt die Lösung der Aufgabe durch eine Wärmebehandlung von im Wesentlichen quaderförmigen Packungen bei gleichzeitigem Bewegen der Packungen um eine nicht parallel zu den Packungskanten verlaufende Raumachse, so dass die stückigen Anteile jeweils auf einer schiefen Ebene liegen, welche ihre Lage ständig ändert.

Vorrichtungsmäßig wird die Aufgabe dadurch gelöst, dass im Inneren des Autoklaven Einrichungen zum Anordnen von im Wesentlichen quaderförmigen Packungen (P) und zum rotatorischen Antrieb der Packungen (P) um eine nicht parallel zu den Packungskanten verlaufende Raumachse vorgesehen sind.

Die Erfindung hat erkannt, dass eine gleichmäßige durch Mischung des stückige Anteile enthaltenden Flüssigkeitsproduktes dadurch erfolgen kann, dass durch geeignete Bewegung der Verpackung die stückigen Anteile jeweils auf einer schiefen Ebene liegen, welche ihre Lage ständig ändert. Durch Bewegung um die Raumachse wird das Produkt größtmöglich umgewälzt. Der Schwerkraft folgend, rutschen die stückigen Anteile im Inneren der Packung jeweils in die tiefstliegende Stelle und bewirken so eine Rührbewegung im Inneren der Packung, wodurch eine gleichmäßige Erwärmung des zu sterilisierenden Gutes in kürzerer Zeit gewährleistet wird. Auf Grund der ständigen Bewegung kommt es weder zu Anbackungen von überhitztem Produkt noch zu Qualitätseinbußen durch nicht ausreichend erwärmte Produktbestandteile oder durch Fleckenbildung auf der Innenseite des Packstoffes. Insbesondere wird auch der typische "Konservengeschmack" vermieden. Ein wesentlicher Vorteil des erfindungsgemäßen Verfahrens bzw. der entsprechenden Vorrichtung liegt darin, dass die Behandlungsdauer auf Grund der ständigen Bewegung innerhalb des Produktes gegenüber einer "statischen Erwärmung" deutlich verringert werden kann. Hier sind Verkürzungen des Behandlungszeitraumes von mehr als 10 % möglich.

Nach einer weiteren Lehre der Erfindung kann das Bewegen der Packung kontinuierlich erfolgen, wobei es denkbar ist, die Bewegung als gleichmäßige Drehbewegung vorzunehmen. Alternativ ist es jedoch auch möglich, dass das Bewegen der Packung diskontinuierlich erfolgt, beispielsweise durch eine Bewegung mit Pausen oder mit einer Richtungsumkehr.

Gemäß einer weiteren Ausbildung der Erfindung ist es möglich, dass die Transportbewegungen der Packungen gleichzeitig als Antrieb der relativen Bewegung der Packung zur Transportrichtung ausgenutzt und so auf einen separaten Antrieb verzichtet werden kann.

Hier sieht eine erste Lehre der Erfindung eine Vorrichtung vor, bei der die Packungen, einzeln oder in kleinen Gruppen, während des Transports durch den Autoklaven um eine Achse bewegt werden, und bei der die Linearbewegung der Packungen auch für deren rotatorischen Antrieb dient.

Hierbei ist es möglich, dass der Bewegungsantrieb über eine spezielle Geometrie der Führungsschienen erfolgt. Unabhängig von der Anzahl der Schienen kann ein jeweils wechselndes Verschwenken um eine horizontale Achse der den Autoklaven durchlaufenden Packungen erreicht werden.

Eine alternative Lehre der Erfindung sieht vor, dass der Bewegungsantrieb über mit den Packungen durch den Autoklaven transportierte Zahnräder erfolgt, die mit einer entlang des Transportweges fest angeordneten Zahnstange oder dergleichen kämmen. Diese Lösung ist insbesondere dann von Vorteil, wenn die gesamten Packungen ständig um sich selbst gedreht werden sollen.

Es ist klar, dass während der Bewegung beim Sterilisierprozess die auf den Trays befindlichen Packungen ihre relative Position zueinander beibehalten müssen, daher sind entsprechende Abstands-, Führungs- oder Klemmelemente vorgesehen. Werden die Packungen eingeklemmt, so erfolgt dies in weiterer Ausgestaltung der Erfindung insbesondere an unbedruckten Stellen der Packungen. So wird eine Beschädigung des auf die Oberfläche des Kartonverbundes aufgebrachten Druckbildes vermieden.

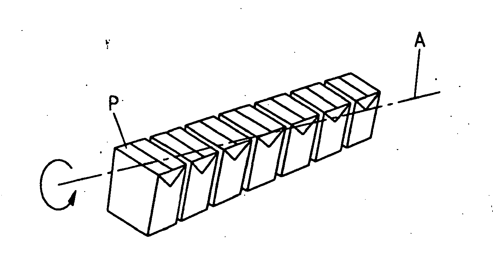

Die Erfindung ist nachfolgend anhand einer lediglich ein bevorzugtes Ausführungsbeispiel darstellenden Zeichnung näher erläutert. In der Zeichnung zeigt die einzige Figur eine Gruppe von zu sterilisierenden Packungen mit gemeinsamer Rotationsachse. Dort ist schematisch eine Gruppe von Packungen P dargestellt, welche, entsprechend beabstandet zueinander angeordnet, entlang einer gemeinsamen Raumachse A rotiert werden können, wobei diese Raumachse A nicht parallel zu den Packungskanten verläuft. Dies führt dazu, dass bei horizontaler Raumachse A in jeder beliebigen Stellung alle Unterseiten stets nicht in der horizontalen Ebene liegen. Auf diese Weise rutschen die stückigen Anteile im Inneren der Packungen P stets an deren tiefste Stelle und bewirken so ein ständiges Durchmischen des Packungsinhaltes.

## Patentansprüche

1. Verfahren zum Sterilisieren eines in einer im Wesentlichen formstabilen Verpackung abgepackten Produktes, wobei die aus Produkt und Verpackung bestehenden Packungen einer Wärmebehandlung bei einer bestimmten Temperatur über eine bestimmte Dauer unterzogen werden, wobei das Produkt aus einer Flüssigkeit und stückigen Anteilen besteht, wobei in einen kontinuierlich arbeitenden Autoklaven eine Mehrzahl von Packungen eingebracht werden und wobei die Packungen nach dem Sterilisierprozess aus dem Autoklaven ausgeschleust werden,
**gekennzeichnet durch**
eine Wärmebehandlung von im Wesentlichen quaderförmigen Packungen bei gleichzeitigem Bewegen der Packungen um eine nicht parallel zu den Packungskanten verlaufende Raumachse, so dass die stückigen Anteile jeweils auf einer schiefen Ebene liegen, welche ihre Lage ständig ändert.

2. Verfahren nach Anspruch 1,
**dadurch gekennzeichnet, dass**
das Bewegen der Packungen kontinuierlich erfolgt.

3. Verfahren nach Anspruch 2,
**dadurch gekennzeichnet, dass**
die Bewegung der Packungen eine Drehbewegung ist.

4. Verfahren nach Anspruch 1,
**dadurch gekennzeichnet, dass**
das Bewegen der Packungen diskontinuierlich erfolgt.

5. Verfahren nach Anspruch 4,
**dadurch gekennzeichnet, dass**
das Bewegen der Packungen mit Pausen erfolgt.

6. Verfahren nach Anspruch 4 oder 5,
**dadurch gekennzeichnet, dass**
das Bewegen der Packungen mit Richtungsumkehr erfolgt.

7. Verfahren nach Anspruch 6,
**dadurch gekennzeichnet, dass**
zum Antrieb der Bewegung der Packungen der Antrieb der Transportbewegung der Packungen verwendet wird.

8. Verfahren nach einem der Ansprüche 1 bis 7,
**dadurch gekennzeichnet, dass**
im Inneren des Autoklaven jede Packung einzeln bewegt wird.

9. Verfahren nach einem der Ansprüche 1 bis 7,
**dadurch gekennzeichnet, dass**
im Inneren des Autoklaven jeweils eines Gruppe von Packungen bewegt wird.

10. Verfahren nach einem der Ansprüche 1 bis 7,
**dadurch gekennzeichnet, dass**
im Inneren des Autoklaven alle im Autoklaven befindlichen Packungen gemeinsam um eine einzige Achse bewegt werden.

11. Vorrichtung zum Sterilisieren eines in einer im Wesentlichen formstabilen Verpackung abgepackten Produktes in einem Autoklaven, für die Durchführung eines Verfahrens nach einem der Ansprüche 1 bis 10, wobei das Produkt aus einer Flüssigkeit und stückigen Anteilen besteht,
**dadurch gekennzeichnet, dass**
im Inneren des Autoklaven Einrichungen zum Anordnen von im Wesentlichen quaderförmigen Packungen (P) und zum rotatorischen Antrieb der Packungen (P) um eine nicht parallel zu den Packungskanten verlaufende Raumachse vorgesehen sind.

12. Vorrichtung nach Anspruch 11,
**dadurch gekennzeichnet, dass**
die Packungen (P), einzeln oder in kleinen Gruppen, während des Transports durch den Autoklaven gedreht werden und dass die Linearbewegung der Packungen auch für deren rotatorischen Antrieb dient.

13. Vorrichtung nach Anspruch 12,
**dadurch gekennzeichnet, dass**
der Bewegungsantrieb über eine spezielle Geometrie von im Inneren des Autoklaven angeordneten Führungsschienen (2) für den Packungstransport erfolgt.

14. Vorrichtung nach Anspruch 13,
**dadurch gekennzeichnet, dass**
der Bewegungsantrieb über mit den Packungen (P) durch den Autoklaven transportierte Zahnräder (3') erfolgt, die mit einer entlang des Transportweges fest angeordneten Zahnstange (2') oder dergleichen kämmen.

15. Verfahren nach einem der Ansprüche 1 bis 10,
**dadurch gekennzeichnet, dass**
jede Packung (P) während des Sterilisierens an unbedruckten Stellen eingespannt ist.

## Claims

1. Process for sterilizing a product, consisting of a liquid and solids, packed in a substantially dimensionally stable packaging, the packages consisting of product and packaging, being subjected to heat treatment at a specified temperature over a specified period, wherein a plurality of packages are inserted into a continuously operating autoclave, and wherein the packages are removed from the autoclave after the sterilization process, **characterized by** heat treatment of substantially square-shaped packages during simultaneous movement of the packages about a spatial axis not running parallel to the package edges, so that the solids in each case lie on an inclined level, which continuously changes their position.

2. Process according to Claim 1, **characterized in that** the movement of the packages takes place continuously.

3. Process according to Claim 2, **characterized in that** the movement of the packages is a rotary movement.

4. Process according to Claim 1, **characterized in that** the movement of the packages takes place discontinuously.

5. Process according to Claim 4, **characterized in that** the movement of the packages takes place with intervals.

6. Process according to Claim 4 or 5, **characterized in that** the movement of the packages takes place with reversal of direction.

7. Process according to Claim 6, **characterized in that** the drive of the transport movement of the packages is used to drive the movement of the packages.

8. Process according to any one of Claims 1 to 7, **characterized in that** each package is moved individually inside the autoclave.

9. Process according to any one of Claims 1 to 7, **characterized in that** in each case a group of packages is moved inside the autoclave.

10. Process according to any one of Claims 1 to 7, **characterized in that** inside the autoclave all packages inserted in the autoclave are moved together about a single axis.

11. Device for sterilizing a product, consisting of a liquid and solids, packed in a substantially dimensionally stable packaging, in an autoclave for executing a process, according to any one of Claims 1 to 10, **characterized in that** devices are provided inside the autoclave for arranging substantially square-shaped packages (P) and for rotary drive of the packages (P) about a spatial axis not running parallel to the package edges.

12. Device according to Claim 11, **characterized in that** the packages (P), individually or in small groups, are turned during transport through the autoclave, and **in that** the linear movement of the packages also serves for their rotary drive.

13. Device according to Claim 12, **characterized in that** the movement drive takes place by means of a special geometry of guide rails (2) arranged inside the autoclave for transporting the packages.

14. Device according to Claim 13, **characterized in that** the movement drive takes place by means of toothed wheels (3'), transported with the packages (P) through the autoclave, which mesh with a toothed rack (2') or the like, firmly arranged along the transport path.

15. Process according to any one of Claims 1 to 10, **characterized in that** during sterilization each package (P) is clamped at plain areas.

## Revendications

1. Procédé pour la stérilisation d'un produit, emballé dans un emballage sensiblement indéformable, le paquet, formé par le produit et l'emballage, étant soumis à un traitement à chaud à une température déterminée, pendant une durée déterminée, le produit consistant en un liquide et en des particules solides, plusieurs paquets étant mis dans un autoclave qui fonctionne en continu, et les paquets étant éclusées en dehors de l'autoclave après le processus de stérilisation, **caractérisé par** un traitement à chaud de paquets, qui présentent sensiblement la forme de parallélépipèdes, avec agitation simultanée autour d'un axe spatial qui s'étend non parallèlement aux bords des paquets de sorte que les particules solides se trouvent respectivement sur un plan oblique qui modifie continuellement sa position.

2. Procédé selon la revendication 1, **caractérisé en ce que** l'agitation des paquets est effectuée en continu.

3. Procédé selon la revendication 2, **caractérisé en ce que** l'agitation des paquets est un mouvement de rotation.

4. Procédé selon la revendication 1, **caractérisé en ce que** l'agitation des paquets est effectuée en discontinu.

5. Procédé selon la revendication 4, **caractérisé en ce que** l'agitation des paquets est effectuée avec des pauses.

6. Procédé selon revendication 4 ou 5, **caractérisé en ce que** l'agitation des paquets est effectuée avec inversion de la direction.

7. Procédé selon la revendication 6, **caractérisé en ce que**, pour l'entraînement des paquets, on utilise l'impulsion du mouvement de transport de paquets.

8. Procédé selon l'une des revendications 1 à 7, **caractérisé en ce que** chaque paquet est mû individuellement à l'intérieur de l'autoclave.

9. Procédé selon l'une des revendications 1 à 7, **caractérisé en ce qu'**un groupe de paquets est respectivement mû à l'intérieur de l'autoclave..

10. Procédé selon l'une des revendications 1 à 7, **caractérisé en ce que** tous les paquets, qui se trouvent dans l'autoclave, sont mus en commun, à l'intérieur de l'autoclave, autour d'un seul axe.

11. Dispositif pour la stérilisation dans un autoclave d'un produit emballé dans un emballage sensiblement indéformable, pour la réalisation d'un procédé selon l'une des revendication 1 à 10, le produit consistant en un liquide et des particules solides, **caractérisé en ce que** des dispositifs sont prévus à l'intérieur de l'autoclave pour ordonner des paquets (P) sensiblement en forme de parallélépipèdes et pour entraîner en rotation des paquets (P) autour d'un axe spatial, qui s'étend non parallèlement aux bords des paquets.

12. Dispositif selon la revendication 11, **caractérisé en ce que** les paquets (P) sont tournés individuellement ou en petits groupes pendant le transport à travers l'autoclave et **en ce que** le mouvement linéaire des paquets sert aussi à leur entraînement en rotation.

13. Dispositif selon la revendication 12, **caractérisé en ce que** l'impulsion du mouvement est effectué au moyen d'une géométrie spéciale de rails de guidage (2), disposés à l'intérieur de l'autoclave pour le transport des paquets.

14. Dispositif selon la revendication 13, **caractérisé en ce que** l'impulsion du mouvement est effectué par l'intermédiaire de roues dentées (3') transportées avec les paquets (P) à travers l'autoclave, qui coopèrent avec une crémaillère (2'), ou un dispositif analogue, disposé fixement le long de la voie de transport.

15. Procédé selon l'une des revendications 1 à 10, **caractérisé en ce que** chaque paquet (P) est maintenu, pendant la stérilisation, à des endroits non imprimés.
